# EUROPEAN PATENT APPLICATION

(11) **EP 3 896 157 A1**
(43) Date of publication of application: **20.10.2021**
(21) Application number: 19896697.0
(22) Date of filing: 11.12.2019
(51) Int. Cl.: C12N 9/24, A21D 8/04

(54) **POLYPEPTIDE WITH XYLANASE ACTIVITY, NUCLEOTIDE SEQUENCE ENCODING IT, INGREDIENT AND PROCESS COMPRISING SAID INGREDIENT FOR PREPARING A FOOD PRODUCT**

(30) Priority: 14.12.2018 CL 20183617
(71) Applicant: Universidad de Santiago de Chile, Santiago (CL)
(72) Inventor: CHÁVEZ ROSALES, Renato Antonio, Estación Central Santiago (CL); GIL DURÁN, Carlos, Estación Central Santiago (CL)
(74) Representative: Herrero & Asociados, S.L.
(86) International application number: PCT/CL2019/050138
(87) International publication number: WO 2020/118465

(57) **Abstract**

The present invention relates to a new polypeptide with xylanase activity, the nucleotide sequence encoding it, an ingredient containing said polypeptide, and a process for preparing a food product which comprises adding said ingredient to the preparation. This new polypeptide with xylanase activity is particularly useful for the baking industry, endowing doughs with greater elasticity, less tenacity, formation of a stronger gluten network with less water, shorter mixing time, and greater volume of the final product.

## Description

### TECHNICAL FIELD

The present invention relates to the technical field of biotechnology, particularly with a new polypeptide having xylanase activity, the nucleotide sequence encoding the same, an ingredient that contains said polypeptide, and a process for the preparation of a food product that comprises adding said ingredient to the preparation.

### BACKGROUND

Xylanases are a class of enzymes that degrade xylan linear polysaccharides to xylose by catalyzing the hydrolysis of the β-(1,4) glycosidic bond. Within this group of enzymes, there are endo-β-(1,4)-D-xylanases (commonly called endo-xylanases) that catalyze the hydrolysis of the xylan internal β-(1,4) glycosidic bond of the xylose chain, generating low-molecular-weight xylooligosaccharides (XOS) with or without branches (Biely, P. Microbial xylanolytic systems. Trends in Biotechnology. 1985. Vol. 3(11): 286-290). These enzymes are classified into different glycoside hydrolase (GH) families according to their physicochemical properties. Mainly, endo-xylanases are classified in the GH10 and GH11 families. The GH10 family xylanases are approximately 35 kDa in size and are capable of hydrolyzing glycosidic bonds close to branch points and towards the non-reducing end, while the GH11 family xylanases are not capable of hydrolyzing said glycosidic bonds and have a smaller size of approximately 20 kDa (Cooper Bribiesca, B. L. Enzimas xilanolíticas bacterianas y sus aplicaciones industriales. VERTIENTES Revista Especializada en Ciencias de la Salud. 2013. Vol. 16(1): 19-22).

Endo-xylanases are of great interest in the food industry. For example, they are widely used in the wine industry, in the production of beer, in the clarification process of fruit juice, in the extraction of starch, coffee and vegetable oils, in the improvement of the nutritional properties of silage and grains, and in bakery industry processes. In particular, for the last industry mentioned, endo-xylanases make it possible to improve a series of characteristics in bakery products, such as the properties of the dough and the volume of the bread. When these enzymes are added during kneading, the viscoelastic properties of the dough are modified, and water absorption, development time of the dough and its stability decrease. As a result, bread and derived products have better characteristics such as volume, firmness, and structure of the crumb (Elgharbi, F., Hmida-Sayari, A., Zaafouri, Y., & Bejar, S. Expression of an Aspergillus niger xylanase in yeast: Application in breadmaking and in vitro digestion. International Journal of Biological Macromolecules. 2015. Vol. 79: 103-109).

In the state of the art there are various xylanases derived from microorganisms or genetically modified, which are mainly focused on the paper industry. For example, patent document US 2015/0291945 A1 refers to an Endo-1,4-β-xylanase from the fungus *Macrophomina phaseolina,* which discloses a series of nucleotide sequences encoding said enzyme. It discloses that these enzymes can be used in the food industry, in the manufacture of animal feed, in pulp and paper production, and in industrial cleaning agents. However, it does not disclose concrete examples of the improvement in their hydrolytic capacity.

On the other hand, patent document US 9,732,366 B2 discloses a high temperature thermostable xylanase of the GH10 family having a catalytic domain including: (A) a polypeptide including a particular amino acid sequence, and (B) a polypeptide including an amino acid sequence in which at least one amino acid has been deleted, substituted, or added in said particular sequence, or (C) an amino acid sequence having at least 80% or greater sequence identity with said particular sequence. The advantage of the xylanase disclosed in US 9,732,366 B2 is that it has activity under conditions of 85°C and at a pH 6.0. However, it does not disclose or exemplify advantages of said enzyme for the food production industry.

Patent document US 2016/0345609 A1 refers to a modified GH10 family xylanase or a fragment thereof having xylanase activity, wherein said mutant has increased thermostability at high temperatures compared with the native enzyme. The modifications correspond to specific mutations at certain positions of the amino acid sequence, which give them stability at temperatures close to 75°C. In turn, patent document WO 2015/114110 A1 discloses synthetic xylanases belonging to the GH10 family having the ability to solubilize insoluble arabinoxylans, particularly from substrates such as corn, wheat, DDGS, etc. This document also focuses on obtaining thermostable xylanases at high temperatures, showing that they are still active in temperature ranges between 61-71°C.

Although the thermostability of xylanase at very high temperatures can be considered as an advantageous characteristic in certain chemical processes, it is completely irrelevant for the processes of food production and in particular for breadmaking, whose fermentation process is carried out at an approximate temperature of 30°C. In this sense, it is more relevant to obtain xylanases whose maximum activity is in a temperature range close to 30°C, and which also provide specific advantages for food manufacturing processes.

### SUMMARY OF THE INVENTION

A first object of the present invention is a polypeptide for preparing a food product, which has xylanase activity, and comprises an amino acid sequence that is selected from the group consisting of:
- the amino acid sequence of SEQ ID No. 1; and
- an amino acid sequence having at least 80% similarity with the sequence of SEQ ID No. 1 and that maintains its xylanase activity.

Said polypeptide is encoded by a nucleotide sequence that is selected from the group consisting of:
- the nucleotide sequence of SEQ ID No. 3; and
- a nucleotide sequence having at least 80% identity with the sequence of SEQ ID No. 3.

A second object of the present invention is a nucleotide sequence encoding a polypeptide having xylanase activity for preparing a food product, wherein said sequence is selected from the group consisting of:
- the nucleotide sequence of SEQ ID No. 3; and
- a nucleotide sequence having at least 80% identity with the sequence of SEQ ID No. 3.

A third object of the present invention is an ingredient for preparing a food product containing a polypeptide having xylanase activity, whose amino acid sequence is selected from the group consisting of:
- the amino acid sequence of SEQ ID No. 1; and
- an amino acid sequence having at least 80% similarity with the sequence of SEQ ID No. 1 and that maintains its xylanase activity.

A fourth object of the present invention is a process for preparing a food product, comprising the steps of:
- providing an ingredient that contains a polypeptide having xylanase activity whose amino acid sequence is selected from the group consisting of:
   - the amino acid sequence of SEQ ID No. 1; and
   - an amino acid sequence having at least 80% similarity to the sequence of SEQ ID No. 1 and that maintains its xylanase activity;
- mixing said ingredient with a flour, a liquid, and any other appropriate ingredient;
- kneading said mixture until a dough is obtained; and
- cooking said dough to obtain a food product.

The flour that is used in the process to prepare a food product is selected from the group consisting of wheat flour, cassava flour, barley flour, rice flour, rye flour, corn flour, quinoa flour, and buckwheat. On the other hand, the liquid that is used to prepare a food product is selected from the group consisting of water, aqueous saline solutions, milk, and infusions.

In a preferred embodiment, the food product obtained from the process described in the present invention is bread.

### BRIEF DESCRIPTION OF THE FIGURES

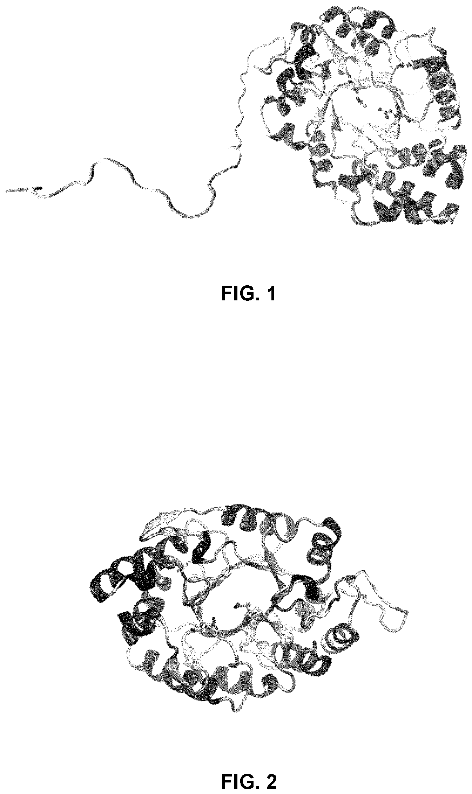

FIG. 1 shows a schematic of the three-dimensional structure of the native enzyme having xylanase activity (XynA) derived from a strain of *Cladosporium* sp.
FIG. 2 shows a schematic of the three-dimensional structure of the modified enzyme having xylanase activity (XynAΔ29N) of SEQ ID No. 1.

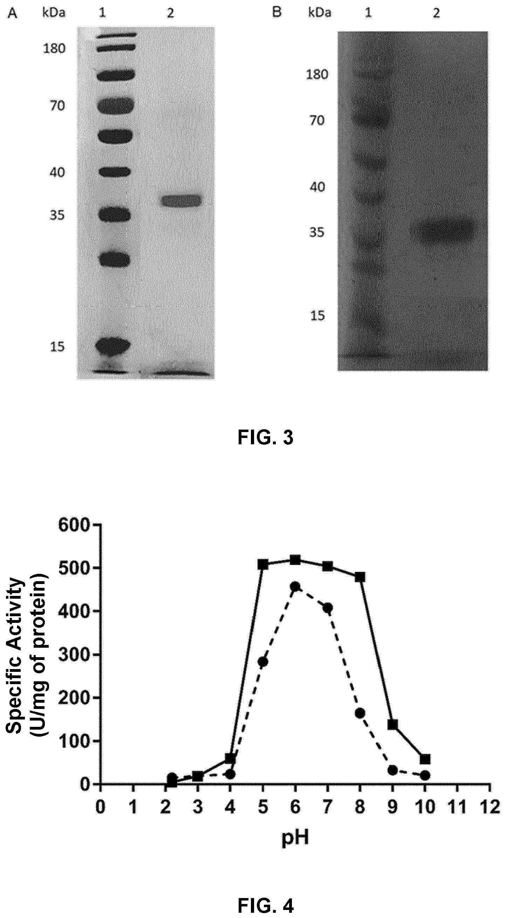

FIG. 3 shows a polyacrylamide gel electrophoresis performed under denaturing conditions of the purified recombinant enzymes. The protein migration profile is shown along with the standard. (A) Lane1. *PageRuler*™ *Prestained Protein Ladder* 10-180 kDA (Thermo Scientific™) with their molecular masses (on the left). Lane 2. Purified XynA. (B) Lane 1. *PageRuler*™ *Prestained Protein Ladder* 10-180 kDA (Thermo Scientific™) with their molecular masses (on the left). Lane 2. Purified XynAΔ29N. The stain used was Coomassie blue.
FIG. 4 shows a graph with the results of the effect of pH on the activity of the enzymes. XynA (dotted line) and XynAΔ29N (solid line). Each of the points represents the average of three replications and the bars the standard deviation.

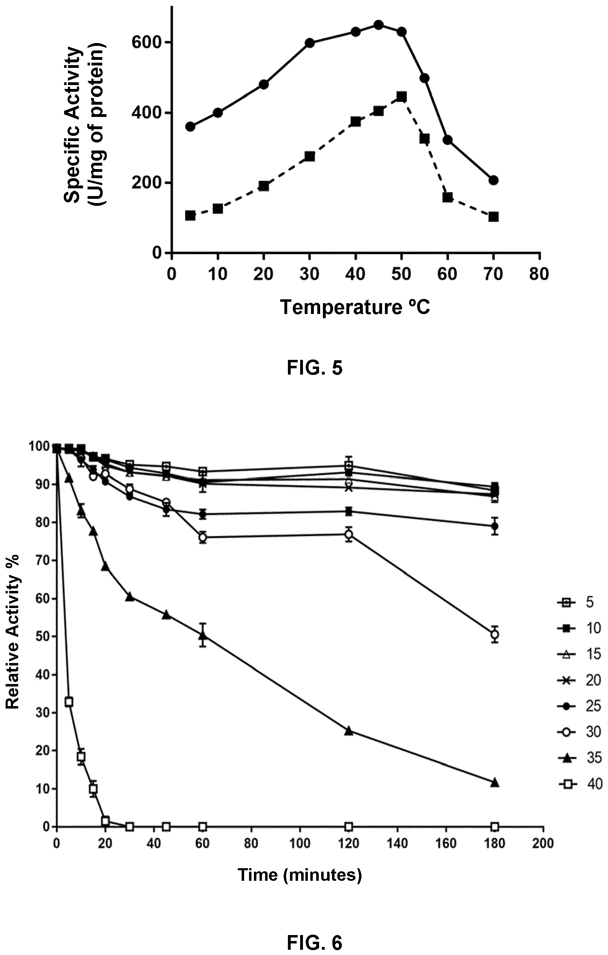

FIG. 5 shows a graph with the results of the specific activity profile of XynA (dotted line) and XynAΔ29N (solid line) at different temperatures. The assays were carried out in triplicate. The bars, corresponding to the standard deviation, were very small, so they are not noticeable in the figure.
FIG. 6 shows a graph with the results of the thermal stability kinetics of XynA. The enzyme was pre-incubated at temperatures between 5 and 40°C. Then, the residual enzyme activity was measured. The enzymatic activity was expressed as a percentage, a 100% being the reaction at time 0 (without pre-incubation). Measurements were made in triplicate and the bars correspond to the standard deviation.

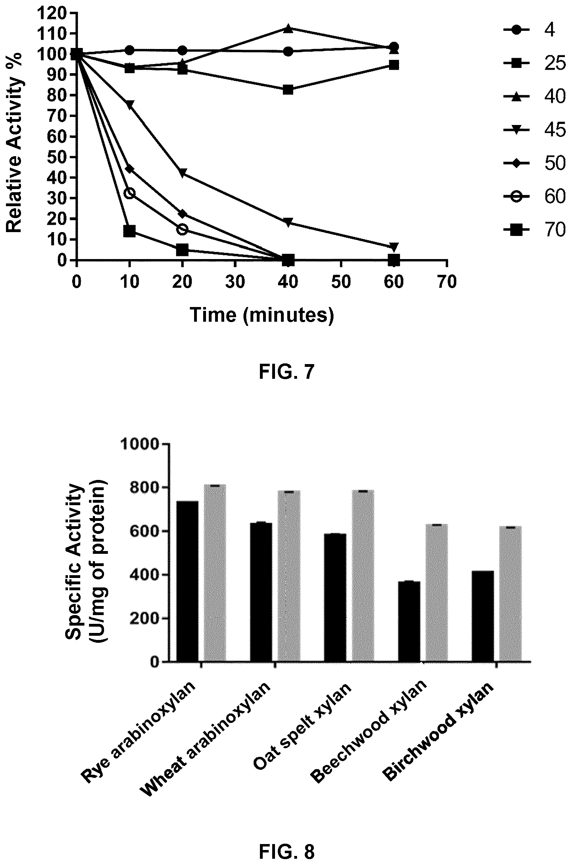

FIG. 7 shows a graph with the results of the thermal stability determination of XynAΔ29N. The enzyme was pre-incubated at temperatures between 5 and 70°C. Then, the residual enzyme activity was measured. The enzymatic activity was expressed as a percentage, a 100% being the reaction at time 0 (without pre-incubation). Measurements were made in triplicate and the bars correspond to the standard deviation.
FIG. 8 shows a graph with the activity of the enzymes on different substrates. The bars correspond to XynA (black color) and XynAΔ29N (gray color). Measurements were made in triplicate and the bars correspond to the standard deviation.

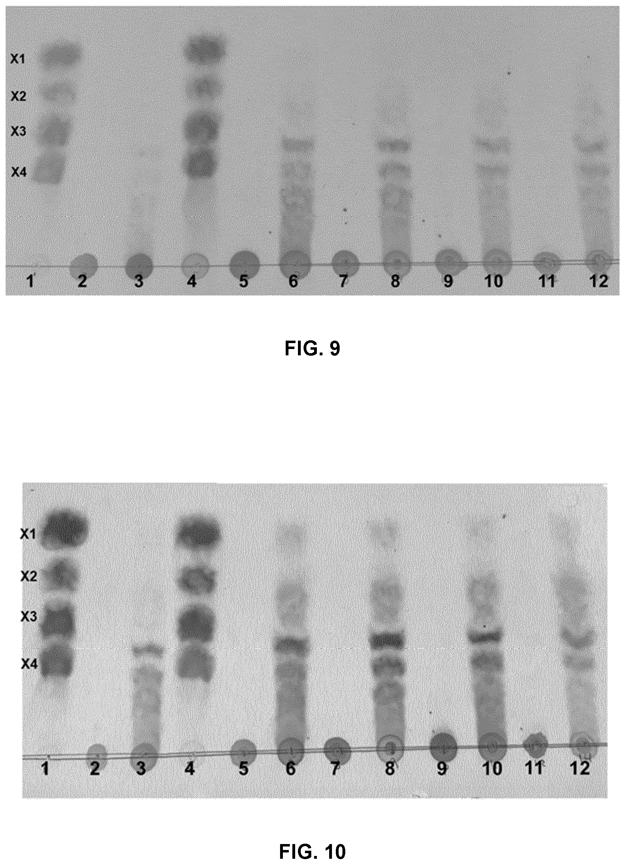

FIG. 9 shows the result of a thin layer chromatography of the hydrolysis products obtained by the action of XynA on xylan. Lanes 1 and 4, standard, mixture of xylooligosaccharides (X1: xylose, X2: xylobiose, X3: xylotriose and X4: xylotetraose); lane 2, rye arabinoxylan; lane 3, rye arabinoxylan plus XynA; lane 5, wheat arabinoxylan; lane 6, wheat arabinoxylan plus XynA; lane 7, oat arabinoxylan; lane 8, oat arabinoxylan plus XynA; lane 9, beech xylan; lane 10, beech xylan plus XynA; lane 11, birch xylan and lane 12, birch xylan plus XynA. Incubation conditions were for 20 min at 50°C.
FIG. 10 shows the result of a thin layer chromatography of the xylan hydrolysis products by XynAΔ29N. Lanes 1 and 4, standard, mixture of xylooligosaccharides (X1: xylose, X2: xylobiose, X3: xylotriose and X4: xylotetraose); lane 2, rye arabinoxylan; lane 3, rye arabinoxylan plus XynA; lane 5, wheat arabinoxylan; lane 6, wheat arabinoxylan plus XynA; lane 7, oat xylan; lane 8, oat xylan plus XynA; lane 9, beech xylan; lane 10, beech xylan plus XynA; lane 11, birch xylan; lane 12, birch xylan plus XynA. Incubation conditions were for 20 min at 50°C. The black box highlights the release of xylose by the XynAΔ29N enzyme.

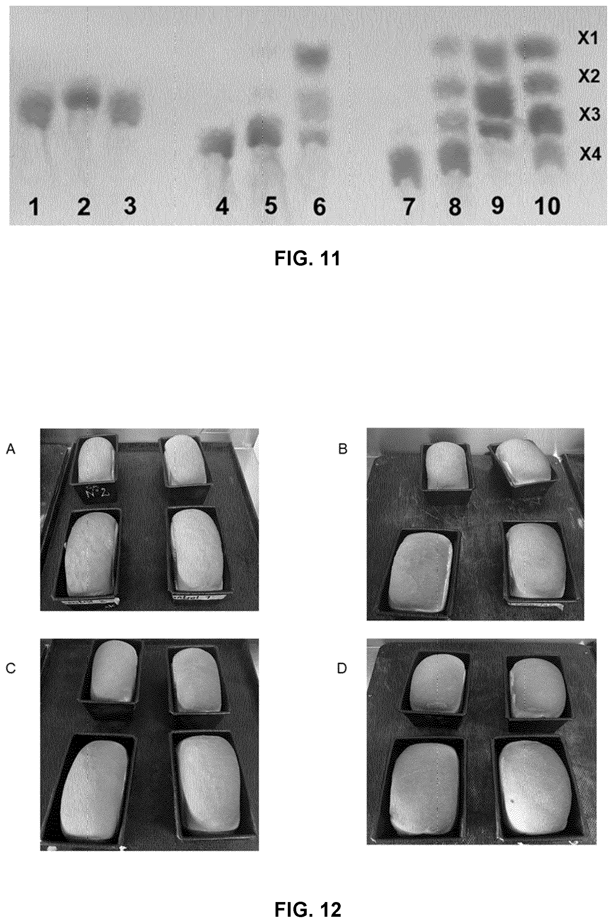

FIG. 11 shows the result of a thin layer chromatography of the hydrolysis products using xylooligosaccharides as substrate. Lane 1, xylobiose; lane 2, xylobiose plus XynA; lane 3, xylobiose plus XynAΔ29N; lane 4, xylotriose; lane 5, xylotriose plus XynA; lane 6, xylotriose plus XynAΔ29N; lane 7, xylotetraose; lane 8, xylotetraose plus XynA; lane 9, xylotetraose plus XynAΔ29N; lane 10, mixture of xylooligosaccharides (X1: xylose, X2: xylobiose, X3: xylotriose and X4: xylotetraose). Incubation conditions were for 20 min at 50°C.

FIG. 12 shows photographs of breads made. (A) Bread without enzyme. (B) Bread made with commercial enzyme. (C) Bread made with the addition of XynA. (D) Bread made with the addition of XynAΔ29N.

### DETAILED DESCRIPTION OF THE INVENTION

The present invention relates to a new polypeptide having xylanase activity, specifically suitable for improving products derived from the food industry, preferably for the bakery industry. By adding this new polypeptide to the mixtures to generate food products derived from the bakery industry, it allows them to have better elasticity and less tenacity, and to obtain a final product of greater volume, compared to polypeptides having xylanase activity currently available on the market.

All technical and scientific terms used to describe the present invention have the same meaning understood by a person with basic knowledge in the technical field in question. However, to define the scope of the invention more clearly, the following is a list of the terminology used in this description and its meaning.

The term "nucleotide sequence" is to be understood as a double strand of DNA, or a single strand of DNA, natural or synthetic, or products of the transcription of said DNA (for example, RNA molecules) . It should be understood that the present invention does not relate to genomic nucleotide sequences in their natural state, but rather refers to nucleotide sequences in an isolated, or purified, or partially purified, or recombinant state, obtained by any method of genetic engineering known in the state of the art.

The term "amino acid sequence" or "polypeptide" is to be understood as an amino acid sequence, natural or synthetic, or products of RNA translation. When these polypeptides have a stable and three-dimensional structure, they are called proteins. It should be understood that the present invention does not relate to amino acid sequences in their natural state, but rather refers to amino acid sequences or polypeptides in an isolated, or purified, or partially purified, or recombinant state, obtained by any method of genetic engineering known in the state of the art.

The term "enzyme" should be understood as a polypeptide that acts as a biological catalyst that accelerates chemical reactions that would otherwise occur but at very low rates. Enzymes convert substrates into different molecules called products. These enzymes have specific affinities for their substrates according to their three-dimensional structure. The biological activity of these molecules is sensitive to environmental conditions, such as pH and temperature, or even other molecules that can increase or inhibit their activity.

The term "xylanase activity" should be understood as the enzymatic activity carried out by a polypeptide that produces the hydrolysis of xylan when this molecule is the substrate.

The term "recombinant" should be understood as any nucleotide (DNA, RNA) or amino acid sequence modified by any genetic engineering method known in the state of the art, which generates as a result a new nucleotide or amino acid sequence different from the one found in nature.

The term "identity" between nucleotide sequences is to be understood as the percentage of identical nucleotides that the compared sequences share with each other, in a particular comparison window. Percent identity can be calculated using a sequence comparison algorithm or by manual alignment and visual inspection. For example, the sequences and identity percentages can be obtained using computer resources available on the Internet such as the BLAST computer program (http://blast.ncbi.nlm.nih.gov/) or the FastDB computer program. Sequence identity can also be determined by hybridization assays. The higher the degrees of hybridization stringency used in the assay, the greater the sequence complementarity required for them to hybridize. High stringency conditions are described by Sambrook et al. (Molecular Cloning A Laboratory Manual, Cold Spring Harbor Press, 1989). In the case of amino acid sequences, the term "similarity" will be used, which includes the percentage of identical amino acids and the percentage of non-identical amino acids that share a high degree of likeness between the compared sequences in a particular comparison window. The amino acids that share a high degree of likeness correspond to those amino acids that belong to the same classification according to the physicochemical characteristics of their side chain (R), and that can be amino acids with polar but uncharged R groups (Ser, Thr, Asn, Gln), with positively charged R groups (Lys, Arg, His), negatively charged R groups (Glu, Asp), hydrophobic R groups (Ala, Ile, Leu, Met, Phe, Trp, Val, Tyr), or special amino acids (Cys , Gly, Pro).

A first object of the present application corresponds to a polypeptide or modified enzyme having xylanase activity, whose amino acid sequence is shown in SEQ ID No. 1. This corresponds to a modified amino acid sequence from a native enzyme having xylanase activity (SEQ ID NO. No. 2, GenBank access code MG007677), which was isolated from the fungus *Cladosporium* sp., obtained from Antarctic marine sponges (Henriquez M, et al. Diversity of cultivable fungi associated with Antarctic marine sponges and screening for their antimicrobial, antitumoral and antioxidant potential. World J Microbiol Biotechnol. 2014. Vol. 30(1):65-76). The modification of the native enzyme was carried out using genetic engineering techniques known in the state of the art. From said enzyme, 29 amino acid residues were deleted from the N-terminal end (amino-terminus), which gives a surprising advantage to the enzyme having xylanase activity claimed in the present invention compared to the native enzyme, significantly improving its activity over several substrates, at different temperatures and pHs.

The modified polypeptide having xylanase activity of the present invention has a specific activity in a wide pH range, its activity being stable and optimal between pH 5 to 8. On the other hand, the polypeptide of the present invention has a specific activity in an optimum temperature range for processes in the food industry, in a range between 30 and 50°C. Within this entire temperature range, the enzyme maintains a stable maximum activity, unlike other enzymes described in the state of the art, which tend to have a maximum peak of activity at a particular temperature and then rapidly decay at temperatures close to said maximum, so the temperature range is limited. This maximum activity in this temperature range is particularly desired for breadmaking processes, the enzyme being active during the kneading stage until the beginning of the cooking stage.

Although a particular deletion of 29 amino acid from the N-terminal end of the native enzyme was chosen, a person skilled in the art would expect that the deletion of a similar number of amino acids from that amino-terminus will produce the same advantageous effect for the enzyme. Consequently, any xylanase enzyme that undergoes progressive deletion of amino acids from the amino terminal end is within the scope of the present invention, and maintains a similarity percentage equal to or greater than 80%. For example, any xylanase enzyme having 29 ± 10 amino acids removed from its amino terminal end is within the scope of the present invention, without said approximate value limiting the scope of the present invention.

In a particular embodiment, the polypeptide having xylanase activity is capable of degrading polysaccharides β-(1,4)-xylan to xylose. Said polypeptide preferably having endo-β-(1,4)-D-xylanase activity, which catalyzes the hydrolysis of the xylan internal β-(1,4) glycosidic bond of the xylose chain. However, the present enzyme having xylanase activity can hydrolyze other substrates such as, xylooligosaccharides (xylotriose, xylotetraose), p-nitrophenyl β-d-xylopyranoside (pNPX), p-nitrophenyl β-D-glucopyranoside (pNPG), p-nitrophenyl-α-L-arabinofuranoside, *p*-nitrophenyl-α-L-arabinopyranoside, *p*-nitrophenyl-β-L-arabinopyranoside, *p*-nitrophenyl-β-D-mannopyranoside, *p*-nitrophenyl-α-D-galactopyranoside, *p*-nitrophenyl-β-D-galactopyranoside, compound glucans composed of β-1,3, β-1,4 and β-1,6 bonds, oligosaccharides composed of β-1,4 and β-1,6 bonds.

On the other hand, it is also within the scope of the present invention any amino acid sequence of the new polypeptide having xylanase activity having at least 80% similarity with the sequence described in SEQ ID No. 1, preferably 90% similarity, more preferably 95% similarity, as long as any of the variants maintain their advantageous xylanase activity. Any person having ordinary skill in the art will recognize that certain amino acids share their physicochemical characteristics depending on their side chain, so the modification of one or more of the amino acids of SEQ ID No. 1 will be irrelevant and will not change the biological activity of said enzyme, reason why said variants are also object of the present invention.

The polypeptide of the present invention can be obtained by any biotechnological technique known in the state of the art for this purpose. For example, the present polypeptide can be obtained by recombinant DNA techniques, expressing a polynucleotide encoding said polypeptide in a suitable expression vector, which is introduced into an appropriate organism for the production of said polypeptide. The production of the polypeptide can be carried out in bioreactors for obtention on a large scale.

A second object of the present invention is a nucleotide sequence encoding the previously described polypeptide having xylanase activity, and that is shown in SEQ ID No. 3. This nucleotide sequence was modified from the nucleotide sequence encoding the native polypeptide having xylanase activity (SEQ ID No. 4, GenBank access code MG007677), which was isolated from the fungus *Cladosporium* sp., obtained from Antarctic marine sponges (Henriquez M, et al. Diversity of cultivable fungi associated with Antarctic marine sponges and screening for their antimicrobial, antitumoral and antioxidant potential. World J Microbiol Biotechnol. 2014. Vol. 30(1):65-76). Although a particular deletion of nucleotides encoding 29 amino acid from the N-terminal end of the native enzyme was chosen, a person skilled in the art would expect that the deletion of a similar number of nucleotides encoding amino acids from that amino-terminus will produce the same advantageous effect for the polypeptide having xylanase activity. Consequently, any nucleotide sequence encoding a polypeptide having xylanase activity that has, for example, 29 ± 5 amino acids removed from its amino terminus, is within the scope of the present invention, without said approximate value limiting the scope of the present invention.

The present invention also considers any variant of said nucleotide sequence having at least 80% identity with the sequence described in SEQ ID No. 3, preferably 90% identity, more preferably 95% identity, as long as any of the variants maintain their ability to encode a polypeptide having xylanase activity. Any person having ordinary skill in the art will recognize that the genetic code is degenerate, and as such the nucleotide sequence can have different codons encoding the same amino acid. Consequently, certain changes in the nucleotide sequence SEQ ID No. 3 may be irrelevant and will not change the biological activity of the enzyme that is encoded by said sequence.

A third object of the present invention is an ingredient for preparing a food product containing the polypeptide having xylanase activity, the amino acid sequence of which is selected from the group consisting of the amino acid sequence of SEQ ID No. 1; and an amino acid sequence having at least 80% similarity to the sequence of SEQ ID No. 1 and that maintains its xylanase activity.

Said ingredient containing the polypeptide is useful to improve the organoleptic characteristics of final food products and/or to improve their characteristics during their manufacturing process. For example, when the present enzyme is added during the breadmaking processes, it is observed that it allows the dough to have a greater elasticity, less tenacity, greater formation of the gluten network with less water, a decrease in kneading time, and a greater volume of the final product. The present enzyme can also be used in the beverage production industry to improve the properties of juices, wines, and beers. In particular, the xylanase of the present invention makes it possible to increase the yield of obtaining fruit juices when it is added to the manufacturing process during the mashing process of said fruits. Furthermore, the present enzyme improves the clarity of juices, wines, and beers, and reduces turbidity, which are characteristics highly desired in these products. These examples should be considered as such, since any person having ordinary skill in the art will recognize that the enzyme of the present invention can be used for multiple industrial food manufacturing processes, without being limited to those previously mentioned.

The composition of this ingredient, in addition to the polypeptide having xylanase activity, may optionally contain excipients suitable for later use in the food industry. Said excipients can be stabilizers (e.g., salts, inert proteins, carbohydrates), preservatives, solvents, diluents, coating, among others, which allow to maintain the stability of the polypeptide over time, increase its half-life in the market, avoid its agglomeration, etc. On the other hand, the ingredient can be in a liquid or solid formulation. It can be formulated as a solution with the polypeptide in suspension, or as a powder, e.g., lyophilized.

A fourth object of the present invention is a process for preparing a food product, comprising the steps of:
- providing an ingredient containing a polypeptide having xylanase activity whose amino acid sequence is selected from the group consisting of:
   - the amino acid sequence of SEQ ID No. 1; and
   - an amino acid sequence having at least 80% similarity to the sequence of SEQ ID No. 1 and that maintains its xylanase activity;
- mixing said ingredient with a flour and a liquid and any other appropriate ingredient;
- kneading said mixture until a dough is obtained; and
- cooking said dough to obtain a food product.

The flour that is used in the process for preparing a food product is any fine powder that is obtained from cereals or ground grains or other foods rich in starch. For example, the flour used can be wheat flour, cassava flour, barley flour, rice flour, rye flour, corn flour, quinoa flour, and buckwheat flour.

On the other hand, the liquid that is used for preparing a food product is selected from the group consisting of water, saline solutions, milk, and infusions, but any other liquid approved for human consumption can also be used. Other appropriate ingredients can optionally be added such as vegetable or animal fat (oils, butters, margarines, shortenings, etc.), flavorings (salts, sugars, spices, etc.), yeasts, baking powders (citric or tartaric acid mixed with sodium carbonate or bicarbonate), sodium bicarbonate, grains and seeds (flaxseed, chia, poppy seeds, pumpkin seeds, oats, sesame, etc.), nuts (almonds, walnuts, peanuts, raisins, etc.), fresh or dehydrated fruits and vegetables, eggs and other proteins, among others.

The kneading of the mixture can be done manually or mechanically, with kneading machines. In certain breadmaking processes, a dough resting stage can be optionally added prior to its cooking.

On the other hand, the cooking of the dough used in the breadmaking processes described in the present invention is preferably baking. However, any other known cooking method can be used such as sautéing, frying, steaming, etc.

In a preferred embodiment, the food product obtained from the process described in the present invention is bread. Said bread can be of any type and should be understood in its broadest meaning, as any dough-derived product whose minimum ingredients are flour and a liquid. It includes sweet or savory, white, whole grain breads, of any shape and cooking. The present invention is not limited to the foregoing, and it is possible to use the enzyme in tortilla preparations, or dough with spongy textures, such as cookies, buns, donuts, kuchen, sponge cakes, cakes, etc.

### EXAMPLES

### Example 1. Isolation of the nucleotide sequence encoding the polypeptide having xylanase activity from Cladosporium sp.

A strain of *Cladosporium* sp. from Antarctic marine sponges (Henriquez M, et al. Diversity of cultivable fungi associated with Antarctic marine sponges and screening for their antimicrobial, antitumoral and antioxidant potential. World J Microbiol Biotechnol. 2014. Vol. 30(1):65-76) was isolated. Total RNA was obtained from said strain according to the protocol briefly described below. In a 250 mL flask containing 100 mL of Czapeck medium with 1% beech xylan, it was inoculated with 5.0 x 10⁶ spores/mL, for 48 hours at 15° C with 180 rpm shaking. Subsequently, the mycelium was filtered and washed 4 times with a DEPC-treated 0.9% NaCl solution. Then, the mycelium was dried and pulverized. RNA extraction was performed with the *RNeasy Plant Mini kit* (Qiagen®) according to the manufacturer's instructions. The RNA obtained was quantified and treated with DNase I - RNase-free, following the manufacturer's instructions. RNA was stored at -80°C until its use.

### Obtaining cDNA from the native xylanase by RT-PCR

In order to obtain the native xylanase gene, RT-PCR was performed. The reverse transcriptase reaction was performed using the *RevertAid Reverse Transcriptase* enzyme (Thermo Scientific™) and oligo (dT) 20 was used to obtain cDNA. Then, a PCR was performed using recombinant Taq polymerase (Invitrogen), where the oligonucleotides used for said PCR were Picz-Xyl-Ecorl-Fw (SEQ ID No. 5) and Picz-Xyl-Sacll-Rv (SEQ ID No. 6). The PCR products were visualized by electrophoresis, purified, and cloned into pGEM®-T Easy (Promega®) for their sequencing.

### Example 2. Heterologous expression of xylanases XynA and XynAΔ29N in Pichia pastoris GS115.

The nucleotide sequence of the native xylanase (XynA SEQ ID No. 4) was obtained. The three-dimensional structure of the polypeptide sequence encoded by SEQ ID No. 4 is shown in FIG. 1, which was modeled by the Modeller® program. This scheme was obtained by XynA homology. In the central part are the glutamate residues that possibly carry out the catalytic activity. It should be noted that the amino terminal end is not modeled by the Modeller® program. As templates for the model, several xylanase crystal structures available from the *Protein Data Bank* (PDB) database were downloaded.

From this polypeptide, a recombinant enzyme was generated in which the 29 non-modeled amino acids located at the amino terminal end were deleted. This new model was called XynAΔ29N, and its three-dimensional structure is presented in FIG. 2. SEQ ID No. 3 shows the nucleotide sequence encoding the modified xylanase of SEQ ID No. 1.

It is important to note that, according to the three-dimensional model, the deletion of the 29 amino acids did not generate an evident change in the general topology compared to the model generated for XynA, since, except for the lack of the 29 amino acids, the structure (α8/β8) is conserved.

The cDNA obtained from RNA was cloned by reverse transcription as previously described, and subsequently amplified by PCR, using the oligonucleotides Picz-Xyl-Ecorl-Fw (SEQ ID No. 5) and Picz- Xyl-SacII-Rv (SEQ ID No. 6) for cDNA-XynA (native xylanase), and oligonucleotides Picz-xylΔ29N-Ecorl fw (SEQ ID No. 7) and Picz-Xyl-Sacll-Rv (SEQ ID No. 6) for cDNA-XynΔ29N (modified xylanase).

Next, the vector pPICZα (obtained from the EasySelect™ Pichia Expression kit, Invitrogen™) and the cDNAs (cDNA-XynA and cDNA-XynΔ29N) were digested using the enzymes EcoRI and SacII. Digestions were carried out separately. For the Sacll enzyme, in a final volume of 20 µL, 2 µL of 10X tango buffer, 2 µL of enzyme (20 Units), 4 µL of DNA at a concentration of 1 µg/µL, and 12 µL of water were added. The digestion was incubated for 2 hours at 37°C, and then the fragments were purified. Subsequently, digestion with the enzyme EcoRI was performed. The same procedure explained for the Sacll digestion was followed, except that the buffer used was buffer O. To improve cloning efficiency, the digested plasmid pPICZα was dephosphorylated, using Antarctic alkaline phosphatase. This reaction was carried out in conjunction with the final digestion with EcoRI, adding 1 µL of Antarctic alkaline phosphatase (10 Units) and 2 µL of the buffer. Finally, all the products were purified.

The fragments obtained after double digestion were ligated to pPICZα (EasySelect™ Pichia Expression kit, Invitrogen™). A vector:insert ratio of 1:3 in 10 µL was used as final volume. In the reaction, 1 µL of "10X ligation buffer", 2 µL of T4 ligase (5 U/µL), 1 µL of vector pPICZα (50 ng/µL), 3 µL of the digested PCR products at a concentration 150 ng/µL, and 4 µL of MiliQ water were added. The reaction was incubated for 16 hours at 15°C.

Subsequently, the bacterial transformation was carried out. After this, the transformants were spread on Low Salt LB agar medium at pH 7.5, supplemented with 0.1 mg/mL Zeocin. The presence of the insert was verified in the colonies obtained after transformation by PCR using the oligonucleotides Xyl RT Fw (SEQ ID No. 5) and Xyl RT Rv (SEQ ID No. 6). The positive transformants were cultured in Low Salt LB liquid medium at pH 7.5 for the subsequent extraction of the plasmid.

### Selection of Pichia pastoris GS115 clones having xylanase activity.

Five clones of P. *pastoris* GS115 resistant to Zeocin were randomly selected to determine whether they had xylanase activity.

Each clone was inoculated in 20 mL of BMGY medium in 250 mL flasks, and incubated for 1 day at 28°C and 200 r.p.m. Then, the culture was centrifuged at 2400 g for 5 minutes at room temperature, and the cells were resuspended in 50 mL of BMMY to induce the expression of the gene of interest with methanol for 8 days. Each day, a 1 mL aliquot of the culture supernatant was taken to test for xylanase activity as described below. For the measurement of xylanolytic activity, reducing sugars were detected by the 3,5-dinitrosalicylic acid (DNS) assay (Bailey, M. J., Biely, P., & Poutanen, K. Interlaboratory testing of methods for assay of xylanase activity. Journal of Biotechnology. 1992. Vol. 23(3): 257-270). 450 µL of a 1% beech xylan solution (substrate) were pre-incubated in 50 mM citrate buffer at pH 5.3 for 10 minutes at 50°C. Then, 50 µL of the samples were added, and they were incubated for 10 minutes (reaction time) at 50°C. The reaction was stopped using 750 µL of DNS reagent (1% 3,5-dinitrosalicylic acid, 30% sodium potassium tartrate, and 1.6% NaOH). Subsequently, it was incubated at 100°C for 10 minutes. Samples were cooled at room temperature, centrifuged at 8600 g to remove residual xylan, and absorbance at 540 nm was measured in the supernatant. For the calibration curve, xylose was used in concentrations from 0 to 20 mM. One unit (U) of enzyme activity was defined as the amount of enzyme necessary to produce 1 µmol of reducing sugars per minute.

### Purification of XynA and XynAΔ29N proteins

For enzymatic purification, 2 liters of the XynA and XynAΔ29N transformants were cultivated in BMMY medium. The cultures were concentrated 200 times (2000 mL to 10 mL), dialyzed in an appropriate buffer, and subjected to chromatography.

For the purification of XynA, fractions having xylanase activity were obtained through chromatography using His Pure Ni-NTA resin (Thermo Scientific™), which were dialyzed and subjected to ion exchange chromatography using DEAE-Sephadex®. Finally, the fractions having xylanase activity from DEAE-Sephadex® chromatography were dialyzed and subjected to chromatography using Sulfopropyl Sepharose® resin. From this last chromatographic step, the xylanase was purified to homogeneity. FIG. 3 shows the SDS-PAGE gel of one of the fractions obtained by this chromatographic step.

For purification of XynAΔ29N, 5 mL was loaded onto the column with His Pure Ni-NTA resin (Thermo Scientific™). Protein was purified to homogeneity with this single chromatographic step, as shown in FIG. 3.

### Example 3. Biochemical characterization of xylanases XynA and XynAΔ29N.

### Effect of pH on enzymatic activity

The results of this experiment are shown in FIG. 4. XynA shows an optimum pH of 6.0, with a specific activity of 457 ± 2.0 U/mg. At pH 5, the specific activity of XynA drops to 283 ± 2.5 U/mg. At pH 4.0, pH 3.0, and pH 2.2, a decrease in specific activity values is observed at 23 ± 2.0, 19 ± 2.0, and 15 ± 8.0 U/mg, respectively. At pH 7.0, the specific activity obtained was 408 ± 8.0 U/mg, at pH 8.0 it decreased to 164 ± 1.0 U/mg, and at pH 9.0 and 10.0 the activity obtained was 32 ± 1.0 and 20 ± 1.0 U/mg.

On the other hand, the optimal pH results for the XynAΔ29N enzyme (FIG. 4) indicate that this mutant enzyme broadened its activity pH range. In the range of pH 5-8, the average specific activity was 504 ± 4.0 U/mg, while at pH 4 its activity was 59 ± 4.0 U/mg. Finally, at pH 9 the decrease in activity is moderate (activity 138 ± 2.0), while at pH 10 an almost total loss of activity is seen.

### Effect of temperature on enzyme activity

The results of the activity analysis at different temperatures for the XynA and XynAΔ29N enzymes are presented in FIG. 5. At 50°C, the maximum activity for the XynA enzyme is observed, with a specific activity of 445 ± 2.0 U/mg. When the temperature was increased to 55°C, the specific activity of the enzyme was 326 ± 6.0 U/mg. At 60°C and 70°C, the specific activity decreases to 158 ± 7.0 U/mg and 103 ± 6.0 U/mg, respectively. When the temperature is lowered to 45°C, the activity obtained is 404 ± 7.0 U/mg, at 40°C the specific activity is 374 ± 4.0 U/mg, and at 30°C, 20°C, and 10°C the specific activity was 274 ± 6.0 U/mg, 190 ± 2.0 U/mg, and 126 ± 2.0 U/mg, respectively. Finally, at 4°C the specific activity of XynA was 106 ± 2.0 U/mg.

On the other hand, the XynAΔ29N enzyme has its maximum activity at 45°C (648 ± 3.0 U/mg). When the temperature was increased to 50°C, the specific activity was 630 ± 1.0 U/mg, while at temperatures of 55°C, 60°C, and 70°C the specific activities obtained were 498 ± 1.0 U/mg, 322 ± 2.0 U/mg, and 207 ± 1.0 U/mg, respectively. At 40°C, the specific activity was 630 ± 1.0 U/mg, while at temperatures of 30°C, 20°C, 10°C a moderate decrease in activity was observed, retaining 600 ± 4.0 U/mg, 478 ± 2.0 U/mg, and 415 ± 13.0 U/mg, respectively. Finally, it is important to note that at temperatures of 4°C, the XynAΔ29N enzyme has an activity of 370 ± 4.0 U/mg, doubling the activity of XynA at the same temperature.

### Evaluation of thermal stability

The determination of thermostability of XynA was carried out in a temperature range between 5-40°C. The results obtained are observed in FIG. 6. The enzyme loses very little activity upon pre-incubation between 5 and 25°C for 3 hours. However, when pre-incubating at 35°C for 1 hour, the enzyme shows a 50% loss of its activity. It is noteworthy that after 20 minutes at 40°C, the enzyme completely loses its activity. This indicates that XynA is a very heat labile enzyme.

FIG. 7 shows the results of the same experiment for the XynAΔ29N enzyme. Surprisingly, the deletion of all 29 amino acids from the amino terminal end dramatically increased the thermal stability of the enzyme. After 60 minutes at 40°C, XynAΔ29N still retains around 100% of its activity, which decreases when incubating at higher temperatures. However, even at the highest temperature tested (70°C) the enzyme still retains detectable activity after 20 minutes of incubation. This result indicates that the deletion of the 29 amino acids at the amino terminal end of the XynA protein results in an increase in its thermal stability.

### Determination of activity using different substrates

For the determination of the activity of the enzymes on different substrates, the conditions used were pH 6.0 for both enzymes, at temperatures of 50°C for XynA and 45°C for XynAΔ29N. The substrates used were rye arabinoxylan, wheat arabinoxylan, oat shell xylan, beech xylan, and birch xylan. The enzyme was incubated with the substrates at 1% and the activity was determined by the following protocol. For the measurement of xylanolytic activity, reducing sugars were detected by the 3,5-dinitrosalicylic acid (DNS) assay (Bailey, M. J., Biely, P., & Poutanen, K. Interlaboratory testing of methods for assay of xylanase activity. Journal of Biotechnology. 1992. Vol. 23(3): 257-270). 450 µL of a 1% beech xylan solution (substrate) were pre-incubated in 50 mM citrate buffer at pH 5.3 for 10 minutes at 50°C. Then, 50 µL of the samples were added and incubated for 10 minutes (reaction time) at 50°C. The reaction was stopped using 750 µL of DNS reagent (1% 3,5-dinitrosalicylic acid, 30% sodium potassium tartrate, and 1.6% NaOH). Subsequently, it was incubated at 100°C for 10 minutes. Samples were cooled at room temperature, centrifuged at 8,600 g to remove residual xylan, and absorbance at 540 nm was measured in the supernatant. For the calibration curve, xylose was used in concentrations from 0 to 20 mM. One unit (U) of enzyme activity was defined as the amount of enzyme necessary to produce 1 µmol of reducing sugars per minute. The results are shown in FIG. 8.

For both enzymes, the highest activity was presented against rye arabinoxylan, followed by wheat arabinoxylan, and a lower activity against birch and beech xylan, which indicates that both enzymes are more active on xylan having a high content of arabinose. Furthermore, and in accordance with the results of FIG. 8, the deletion of the 29 amino acids increased the specific activity of XynAΔ29N with respect to XynA, in some cases (as in beech xylan) doubling it. This is consistent with what was previously observed in FIG. 5.

### Determination of hydrolysis products generated by XynA and XynAΔ29N from different substrates

To determine the hydrolysis products generated by the action of the enzymes on the different substrates, the technique of thin layer chromatography was used. The results for XynA (FIG. 9) show that the enzyme generates xylooligosaccharides of different lengths. The main hydrolysis products observed were xylobiose, xylotriose, and xylotetraose, but no release of the monosaccharide xylose was observed. These results indicate that XynA is a classical endo-xylanase.

The results of this experiment for the XynAΔ29N enzyme are shown in FIG. 10. XynAΔ29N also generates xylooligosaccharides of different lengths, but in this case the release of xylose was also observed, which suggests that the deletion of the 29 amino acids at the amino terminal end of the protein produces a change in the action of the enzyme on the substrate, transforming the xylanase into a bifunctional endo-xylanase/xylohydrolase enzyme.

### Determination of hydrolysis products generated by XynA and XynAΔ29N from linear xylooligosaccharides

In addition to xylan, enzymatic reactions were performed with linear xylooligosaccharides and the products were analyzed by thin layer chromatography. The results are shown in FIG. 11. Xylobiose is not hydrolyzed by either enzyme. In the case of xylotriose, minimal hydrolysis by XynA is observed. In the case of XynAΔ29N, complete hydrolysis of xylotetraose is observed, with increased xylose production. In the case of xylotetraose, it is observed that both XynA and XynAΔ29N hydrolyze it, generating xylotriose, xylobiose, and xylose, but XynAΔ29N seems to have a higher hydrolytic activity on this substrate, generating xylobiose and xylose as major products.

### Example 4. Application of XynA and XynAΔ29N in breadmaking

For these experiments, in addition to the enzyme produced in the present invention, a commercial fungal xylanase (DIESTRO XF 6000, from Girona SA, Chile) was used, whose dosage recommended by the manufacturer is 2 grams per 100 Kg of flour (20 ppm). Therefore, for the breadmaking experiments, the native enzyme (XynA) and its mutant version (XynAΔ29N) were used in the same dosage.

### Effect of XynA and XynAΔ29N on the rheological characteristics of bread dough

The effect of the XynA and XynAΔ29N enzymes on the dough was evaluated by means of an alveograph analysis. For the measurement of the rheological characteristics of the dough, an alveograph (Chopin, France) was used. This alveograph is composed of three elements: a mixer for preparing the dough, the resting chamber, and the curve recorder (standard manometer).

For the preparation of the dough in the mixer, 250 grams of flour, 138 mL of sodium chloride solution (2.5%), and xylanase (20 ppm) were added. Then, kneading was performed, mixing for a minute, stopping the machine, and separating with a spatula the dough stuck to the walls of the mixer. Subsequently, it was mixed for six more minutes. The first two centimeters of dough were cut off and discarded. The remaining sample was cut into five pieces and rolled over. Once the thickness of the samples had been standardized, they were cut with a circular mold, placing the five pieces of dough in the resting chamber of the alveograph and allowing them rest for 20 minutes at 25°C. After this time, the first sample was placed on the plate to allow the formation of bubbles. Subsequently, the hydrostatic valve was opened, and air was allowed to pass into the dough, which shapes into a balloon that progressively grows until it ruptures. This last step was repeated with the remaining four samples. When opening the hydrostatic valve, the register drew a diagram. Once the five diagrams had been drawn, the average of the analyzed samples was obtained and the tenacity (P, corresponding to the aptitude of the dough to resist deformation) and the extensibility (L, corresponding to the maximum volume of air that the bubble of dough is able to contain; Method 54-30A, International AACC 2000).

From the curves obtained in the diagram, the tenacity (P) and extensibility (L) values were calculated. These calculated values are shown in Table 1. In said table, each value corresponds to the average of three replicates and their standard deviation. Same superscripts (a, b, c) mean that there are no statistically significant differences (Tukey's test, 0.05 significance level).

**Table 1. Effect of xylanases on the tenacity and elasticity of the dough.**

| Treatment | P Value mm (Tenacity) | Tenacity (%)* | L Value mm (Elasticity) | Elasticity (%)* |
|---|---|---|---|---|
| No enzymes | 79.2 ± 1.1^{a} | 100 | 52.0 ± 1.0^{a} | 100 |
| Commercial Xylanase | 73.3 ± 1.6^{b} | 92.6 | 56.3 ±2.1^{b} | 108.2 |
| Xyn A | 72.2 ± 2.5^{b} | 91.2 | 57.7 ± 0.6^{b} | 110.1 |
| XynAΔ29N | 65.2 ± 2.5^{c} | 82.3 | 62.7 ± 2.3^{c} | 120.56 |

| | | | | |
|---|---|---|---|---|
| (*) The treatment without enzymes was established as 100%. | | | | |

The tenacity (P) evaluates the resistance to deformation of the dough. The results of Table 1 indicate that the addition of xylanases to the flour leads to a decrease in the value of this parameter, indicating a decrease in rigidity and in the formation of the gluten network. Specifically, the results show that XynA decreases the tenacity of the dough by 8.8%, while the treatment with XynAΔ29N decreases the tenacity by about 18%, a significant difference compared to the other treatments.

On the other hand, elasticity (L) measures the ability of flour to be stretched once it is mixed with water. The action of xylanases on the doughs generated a change in elasticity, which is reflected in an increase in this parameter (Table 1). All the treatments showed an increase in elasticity, in the case of XynA it increases about 10%, similar to that obtained with the commercial enzyme. In the case of treatment with XynAΔ29N, the elasticity of the dough increased by 21%.

Consequently, the commercial enzyme and the XynA enzyme exhibit similar characteristics in the improvements of the elasticity and tenacity of the doughs, but the XynAΔ29N enzyme exhibits superior characteristics in these parameters. A possible explanation for this phenomenon is the temperatures at which the kneading processes are performed (30°C). At this temperature, XynAΔ29N is much more active than XynA, as the mutant enzyme retains 90% of its activity, while the native enzyme XynA retains 50% of the activity. Another important factor is the hydrolytic profile of the XynAΔ29N enzyme. According to these results (FIG. 8), XynAΔ29N has a higher activity on xylanes having a high content of arabinose, similar to those found in flours. The results presented suggest that the decrease in insoluble arabinoxylans in flour, thanks to the action of XynAΔ29N, led to changes in the interactions between proteins, water, and the other components of the dough, making it more extensible.

### Effect of XynA and XynAΔ29N on the farinographic characteristics of flours

Brabender farinograms are standard curves that allow observing the amount of water that flour needs in order to obtain the ideal consistency, the adequate kneading time to obtain correctly developed dough, and the stability of the dough. Therefore, this experiment made it possible to determine water absorption, dough development time, and stability.

To evaluate the behavior of the dough during kneading, a Brabender® Farinograph (C.W. Brabender Instruments, Inc., Germany) was used. To do this, 50 grams of flour, 30 mL of water, and xylanase (20 ppm) were added to the mixer of the equipment. The mixture was kneaded at a constant speed of 100 r.p.m., recording the resistance that the dough opposes to the continuous mechanical work as a function of time. First, the amount of water necessary for the dough to reach a previously determined consistency of 500 Brabender® units (standardized value of maximum consistency) was determined. Then, from the diagram obtained from the farinograph, the parameters related to the industrial suitability of the flour were obtained.

The development time of the doughs was calculated, as well as their stability. The results are presented in Table 2.

**Table 2. Effect of the addition of xylanases on farinographic parameters.**

| Treatment | | Water added (ml) | Development time | Stability |
|---|---|---|---|---|
| No enzymes | 1 | 31 | 2 min | 10 min 30 sec |
| | 2 | 31 | 2 min | 10 min |
| | 3 | 31 | 2 min | 10 min |
| Commercial Xylanase | 1 | 31 | 2 min | 9 min 30 sec |
| | 2 | 31 | 2 min | 9 min 30 sec |
| | 3 | 31 | 1 min 45 sec | 9 min 30 sec |
| XynA | 1 | 31 | 1 min 30 sec | 9 min 30 sec |
| | 2 | 31 | 1 min 30 sec | 9 min 30 sec |
| | 3 | 31 | 1 min 30 sec | 9 min 30 sec |
| XynAΔ29N | 1 | 30 | 1 min 30 sec | 7 min |
| | 2 | 30 | 1 min 30 sec | 7 min |
| | 3 | 30 | 1 min 30 sec | 7 min |

The results show that by adding the commercial enzyme and the XynA enzyme, a significant decrease in the development time and tolerance of the dough to over-kneading was promoted. The results were more drastic when the XynAΔ29N enzyme was added. With this treatment, a smaller volume of water was required in the mix to generate a consistency of 500 Brabender®. Similarly, kneading tolerance significantly decreased and the development of the gluten network was maintained as in all enzymatic treatments.

### Determination of volume and specific volume of bread

The breads were made using the following formulation: 200 grams of wheat flour (Molino Puente Alto), 8 grams of skimmed milk powder (Svelty®, Nestle), 6 grams of shortening (Astra®), 6 grams of fresh yeast (Lefersa®), 4 grams of sodium chloride (Merck®), 8 grams of common sugar (IANSA®), and 112 mL of water. For the enzyme treatments, these were added at 20 ppm. The ingredients were mixed and kneaded for 7 min with a kneader (Kitchenaid®). Subsequently, the resulting dough was pre-fermented for 105 min at 30°C, in a chamber with an atmosphere of 96% relative humidity. Then, the dough was manually degassed and returned to the resting chamber for another 50 minutes under the same conditions. After this time, the degassed mass was molded and portioned into 125-gram pieces with the help of a spatula. The dough pieces were placed in molds for a final fermentation of 25 min. Finally, the pieces were baked at 215°C for 25 min in a gas oven (Maigas®). Each breadmaking assay was carried out in triplicate.

The measurement of the volume of the bread was determined by displacement of canola seeds, after 1 h of baking. The specific volume was obtained by dividing the volume of the sample by its weight. FIG. 12 shows the result of this bread making.

One hour after the baking is over, the volume, weight, and specific volume of the loaves (volume/weight ratio) were determined. The results are presented in Table 3. In said table, the columns correspond to the average of three replications and the bars to their standard deviation. Same superscripts (a, b, c) mean that there are no statistically significant differences.

**Table 3. Effect of xylanases on the absolute volume, weight, and specific volume of bread.**

| Treatment | Volume (cm³) | Volume (%)* | Weight (gr) | Specific volume (cm³) | Specific volume (%) |
|---|---|---|---|---|---|
| No enzymes | 655 ± 5.0^{a} | 100.0 | 144.5 ± 1.1 | 4.5 ± 0.06^{a} | 100 |
| Commercial Xylanase | 685 ± 5.0^{b} | 104.6 | 148.9 ± 1.2 | 4.6 ± 0.01^{b} | 102.2 |
| XynA | 682 ± 3.0^{b} | 104.1 | 144.3 ± 8.4 | 4.73 ± 0.3^{b} | 105.1 |
| XynAΔ29N | 695 ± 0.0^{c} | 106.1 | 140.8 ± 0.5 | 4.93 ± 0.01^{c} | 109.5 |

| | | | | | |
|---|---|---|---|---|---|
| (*) The treatment without enzymes was established as 100%. | | | | | |

The results in Table 3 indicate that the addition of the xylanases caused a significant increase in bread volume. The XynA enzyme and the commercial enzyme produced a similar increase (4%) in the volume of the bread. On the other hand, in the breads treated with the XynAΔ29N enzyme, a greater volume of 6.1% was observed in relation to the control and 2% more when compared with the commercial xylanase and XynA. A greater difference was also obtained in the specific volume of the bread, since the treatment with XynAΔ29N increases about 10% more compared to the bread that was not treated with enzyme, and 2% and 5% with the commercial xylanase and XynA, respectively, thus producing larger and lighter pieces.

In conclusion, the use of modified XynAΔ29N xylanase of the present invention improves the alveographic parameters (tenacity and elasticity) of the doughs, which allows obtaining loaves with a higher specific volume when compared to the native enzyme and the commercial enzyme.

## Claims

1. A polypeptide for preparing a food product, **CHARACTERIZED in that** it has xylanase activity and comprises an amino acid sequence that is selected from the group consisting of:
- the amino acid sequence of SEQ ID No. 1; and
- an amino acid sequence having at least 80% similarity with the sequence of SEQ ID No. 1 and that maintains its xylanase activity.

2. The polypeptide of claim 1, **CHARACTERIZED in that** the amino acid sequence is encoded by a nucleotide sequence that is selected from the group consisting of:
- the nucleotide sequence of SEQ ID No. 3; and
- a nucleotide sequence having at least 80% identity with the sequence of SEQ ID No. 3

3. A nucleotide sequence encoding a polypeptide having xylanase activity for preparing a food product, **CHARACTERIZED in that** said sequence is selected from the group consisting of:
- the nucleotide sequence of SEQ ID No. 3; and
- a nucleotide sequence having at least 80% identity with the sequence of SEQ ID No. 3.

4. An ingredient for preparing a food product, **CHARACTERIZED in that** it contains a polypeptide having xylanase activity, whose amino acid sequence is selected from the group consisting of:
- the amino acid sequence of SEQ ID No. 1; and
- an amino acid sequence having at least 80% similarity with the sequence of SEQ ID No. 1 and that maintains its xylanase activity.

5. A process for preparing a food product, **CHARACTERIZED in that** it comprises the steps of:
- providing an ingredient that contains a polypeptide having xylanase activity, whose amino acid sequence is selected from the group consisting of:
• the amino acid sequence of SEQ ID No. 1; and
• an amino acid sequence having at least 80% similarity to the sequence of SEQ ID No. 1 and that maintains its xylanase activity;
- mixing said ingredient with a flour, a liquid, and any other appropriate ingredient;
- kneading said mixture until a dough is obtained; and
- cooking said dough to obtain a food product.

6. The process of claim 5, **CHARACTERIZED in that** said flour is selected from the group consisting of wheat flour, cassava flour, barley flour, rice flour, rye flour, corn flour, quinoa flour, and buckwheat.

7. The process of claim 5, **CHARACTERIZED in that** said liquid is selected from the group consisting of water, saline solutions, milk, and infusions.

8. The process of claims 5 to 7, **CHARACTERIZED in that** said food product is bread.


SEQUENCE LISTING

<110>   UNIVERSIDAD DE SANTIAGO DE CHILE

<120>   POLYPEPTIDE WITH XYLANASE ACTIVITY, NUCLEOTIDE SEQUENCE ENCODING IT,
INGREDIENT AND PROCESS COMPRISING SAID INGREDIENT FOR PREPARING A FOOD
PRODUCT

<130>  38-969-RED

<160>  7

<170>  PatentIn version 3.5

<210>  1
<211>  298
<212>  PRT
<213>  Artificial Sequence

<220>
<223>  Modified sequence of the amino acid sequence of a native xylanase
from Cladosporium sp.

<400>  1

Asp Ala Gly Gly Leu Asn Ser Leu Phe Val Ala Lys Gly Lys Glu Tyr
1               5                   10                  15

Phe Gly Thr Cys Ala Asp Pro Gly Thr Leu Ser Ser Ser Glu Thr Ala
            20                  25                  30

Asp Ile Ile Lys Ala Asp Phe Gly Gln Ile Thr Pro Glu Asn Ser Met
        35                  40                  45

Lys Trp Asp Ala Leu Glu Ala Thr Arg Gly Val Phe Thr Phe Asp Thr
    50                  55                  60

Ala Asp Gln Thr Ala Asp Phe Ala Ala Glu Asn Gly Lys Met Leu Arg
65                  70                  75                  80

Cys His Thr Leu Leu Trp His Ser Gln Leu Pro Thr Trp Val Glu Gly
                85                  90                  95

Ile Thr Asp Lys Ala Glu Leu Thr Glu Val Ile Glu Asn His Ile Ala
            100                 105                 110

Glu Val Ala Gly His Phe Lys Gly Lys Cys Phe Ala Trp Asp Val Val
        115                 120                 125

Asn Glu Ile Phe Ala Glu Asp Gly Thr Leu Arg Asp Ser Val Phe Ser
    130                 135                 140

Arg Val Leu Gly Glu Asp Phe Val Ser Ile Ala Phe Glu Ala Ala Lys
145                 150                 155                 160

Lys Ala Ala Pro Asp Ala Lys Arg Tyr Ile Asn Asp Tyr Asn Leu Asp
                165                 170                 175

Ser Ala Thr Tyr Pro Lys Leu Thr Gly Leu Ala Ala Lys Val Lys Glu
            180                 185                 190

Trp Ile Ala Ala Gly Val Pro Ile Asp Gly Ile Gly Ser Gln Ser His
        195                 200                 205

Leu Ser Glu Ser Ser Gly Thr Ala Asp Ala Leu Lys Ala Leu Ala Ala
    210                 215                 220

Ala Ala Pro Glu Val Ala Ile Thr Glu Leu Asp Ile Ala Gly Ala Ser
225                 230                 235                 240

Ala Ala Asp Tyr Glu Ala Val Thr Gln Ala Cys Leu Asp Leu Glu Asn
                245                 250                 255

Cys Val Gly Ile Thr Val Trp Gly Val Ser Asp Ser Gln Ser Trp Arg
            260                 265                 270

Pro Asn Glu Asn Pro Leu Leu Phe Asp Gly Ser Phe Gln Pro Lys Glu
        275                 280                 285

Ala Tyr Thr Gly Leu Thr Thr Leu Leu Ala
    290                 295

<210>  2
<211>  327
<212>  PRT
<213>  Cladosporium sp.

<300>
<301>  Carlos Gil-Durán, María-Cristina Ravanal, Pamela Ubilla,
       Inmaculada Vaca, Renato Chávez
<302>  Heterologous expression, purification and characterization of a
       highly thermolabile endoxylanase from the Antarctic fungus
       Cladosporium sp.
<303>  Fungal Biology
<304>  122
<305>  9
<306>  875-882
<307>  2018-09
<308>  GenBank MG007677
<309>  2017-09-26
<313>  (22)..(348)

<400>  2

Leu Thr Thr Gly Thr Ser Pro Glu Ala Ser Ala Ser Pro Ala Ala Gly
1               5                   10                  15

Ser Ser Asp Ser Ala Ala Gly Gln Thr Gly Glu Ser Ser Asp Ala Gly
                20                  25                  30

Gly Leu Asn Ser Leu Phe Val Ala Lys Gly Lys Glu Tyr Phe Gly Thr
            35                  40                  45

Cys Ala Asp Pro Gly Thr Leu Ser Ser Ser Glu Thr Ala Asp Ile Ile
    50                  55                  60

Lys Ala Asp Phe Gly Gln Ile Thr Pro Glu Asn Ser Met Lys Trp Asp
65                  70                  75                  80

Ala Leu Glu Ala Thr Arg Gly Val Phe Thr Phe Asp Thr Ala Asp Gln
                85                  90                  95

Thr Ala Asp Phe Ala Ala Glu Asn Gly Lys Met Leu Arg Cys His Thr
                100                 105                 110

Leu Leu Trp His Ser Gln Leu Pro Thr Trp Val Glu Gly Ile Thr Asp
            115                 120                 125

Lys Ala Glu Leu Thr Glu Val Ile Glu Asn His Ile Ala Glu Val Ala
    130                 135                 140

Gly His Phe Lys Gly Lys Cys Phe Ala Trp Asp Val Val Asn Glu Ile
145                 150                 155                 160

Phe Ala Glu Asp Gly Thr Leu Arg Asp Ser Val Phe Ser Arg Val Leu
                165                 170                 175

Gly Glu Asp Phe Val Ser Ile Ala Phe Glu Ala Ala Lys Lys Ala Ala
                180                 185                 190

Pro Asp Ala Lys Arg Tyr Ile Asn Asp Tyr Asn Leu Asp Ser Ala Thr
            195                 200                 205

Tyr Pro Lys Leu Thr Gly Leu Ala Ala Lys Val Lys Glu Trp Ile Ala
    210                 215                 220

Ala Gly Val Pro Ile Asp Gly Ile Gly Ser Gln Ser His Leu Ser Glu
225                 230                 235                 240

Ser Ser Gly Thr Ala Asp Ala Leu Lys Ala Leu Ala Ala Ala Pro
                245                 250                 255

Glu Val Ala Ile Thr Glu Leu Asp Ile Ala Gly Ala Ser Ala Ala Asp
                260                 265                 270

Tyr Glu Ala Val Thr Gln Ala Cys Leu Asp Leu Glu Asn Cys Val Gly
      275                 280                 285

Ile Thr Val Trp Gly Val Ser Asp Ser Gln Ser Trp Arg Pro Asn Glu
    290                 295                 300

Asn Pro Leu Leu Phe Asp Gly Ser Phe Gln Pro Lys Glu Ala Tyr Thr
305                 310                 315                 320

Gly Leu Thr Thr Leu Leu Ala
                325

<210>  3
<211>  894
<212>  ADN
<213>  Artificial Sequence

<220>
<223>  Modified sequence of the nucleotide sequence encoding a native
xylanase from Cladosporium sp.

<400>  3
gacgctggag gcctcaacag cttgttcgtc gccaagggca aggagtactt cggtacctgc      60

gccgaccccg gcaccctcag ctcgtctgag accgccgata tcatcaaggc tgacttcggt     120

caaatcaccc cggagaacag catgaagtgg gacgccctcg aggccacccg tggcgtcttc     180

accttcgata cggccgacca gactgccgac tttgctgccg agaacggcaa gatgcttcgc     240

tgccacacgc ttctgtggca ctctcaattg cccacctggg ttgagggcat caccgacaag     300

gctgagctta ctgaggtgat cgagaaccac attgctgagg tcgctggcca cttcaagggc     360

aagtgcttcg cttgggacgt cgtcaacgag atcttcgccg aggatggtac cctccgtgac     420

tctgtctcct cccgcgtcct gggcgaggac ttcgtcagca tcgctttcga ggccgccaag     480

aaggccgctc ctgatgctaa gcgttacatc aacgactaca accttgactc tgccacctac     540

cccaagctta ccggtcttgc cgccaaggtt aaggagtgga tcgccgctgg tgttcccatc     600

gatggcattg gctcccagtc tcacttgccc gagtccagcg gaactgccga cgccctcaag     660

gctcttgctg ccgctgcacc tgaagttgcc atcactgagc tcgacatcgc cggtgcttcc     720

gctgccgact acgaggccgt cacccaggct tgcttggacc tcgagaactg cgttggaatt     780

accgtctggg gtgtttccga ctcccagtca tggaggccca acgagaaccc cctcctcttc     840

gacggaagct tccagccgaa ggaggcctac actggtctca ccaccctcct cgca           894

<210>  4
<211>  981
<212>  ADN
<213>  Cladosporium sp.

<300>
<301>  Carlos Gil-Durán, María-Cristina Ravanal, Pamela Ubilla,
       Inmaculada Vaca, Renato Chávez
<302>  Heterologous expression, purification and characterization of a
       highly thermolabile endoxylanase from the Antarctic fungus
       Cladosporium sp.
<303>  Fungal Biology
<304>  122
<305>  9
<306>  875-882
<307>  2018-09
<308>  GenBank
<309>  2017-09-26
<313>  (64)..(1044)

<400>  4
   ctcaccactg gcacctcgcc tgaggcctcg gcctcgcctg ctgctggttc ttccgactcg       60
   gctgctggcc agactggcga atcttccgac gctggaggcc tcaacagctt gttcgtcgcc      120
   aagggcaagg agtacttcgg tacctgcgcc gaccccggca ccctcagctc gtctgagacc      180
   gccgatatca tcaaggctga cttcggtcaa atcaccccgg agaacagcat gaagtgggac      240
   gccctcgagg ccacccgtgg cgtcttcacc ttcgatacgg ccgaccagac tgccgacttt      300
   gctgccgaga acggcaagat gcttcgctgc cacacgcttc tgtggcactc tcaattgccc      360
   acctgggttg agggcatcac cgacaaggct gagcttactg aggtgatcga gaaccacatt      420
   gctgaggtcg ctggccactt caagggcaag tgcttcgctt gggacgtcgt caacgagatc      480
   ttcgccgagg atggtaccct ccgtgactct gtctcctccc gcgtcctggg cgaggacttc      540
   gtcagcatcg ctttcgaggc cgccaagaag gccgctcctg atgctaagcg ttacatcaac      600
   gactacaacc ttgactctgc cacctacccc aagcttaccg gtcttgccgc caaggttaag      660
   gagtggatcg ccgctggtgt tcccatcgat ggcattggct cccagtctca cttgcccgag      720
   tccagcggaa ctgccgacgc cctcaaggct cttgctgccg ctgcacctga agttgccatc      780
   actgagctcg acatcgccgg tgcttccgct gccgactacg aggccgtcac ccaggcttgc      840
   ttggacctcg agaactgcgt tggaattacc gtctggggtg tttccgactc ccagtcatgg      900
   aggcccaacg agaaccccct cctcttcgac ggaagcttcc agccgaagga ggcctacact      960
   ggtctcacca ccctcctcgc a                                                981


<210>  5
<211>  32
<212>  ADN
<213>  Artificial Sequence

<220>
<223>  Picz-Xyl-EcorI-Fw oligonucleotide

<400>  5
tgaagctgaa ttcctcacca ctggcacctc gc                                     32

<210>  6
<211>  31
<212>  ADN
<213>  Artificial Sequence

<220>
<223>  Picz-Xyl-SacII-Rv oligonucleotide

<400>  6
agactcccgc ggctgcgagg agggtggtga g                                    31


<210>  7
<211>  31
<212>  ADN
<213>  Artificial Sequence

<220>
<223>  Picz-xyl(delta)29N-EcorI fw oligonucleotide

<400>  7
agactccgaa ttcgacgctg gaggcctcaa c                                    31